# EUROPEAN PATENT SPECIFICATION

(11) **EP 4 618 863 B1**
(45) Date of publication and mention of the grant of the patent: **23.09.2026**
(21) Application number: 23825634.1
(22) Date of filing: 05.12.2023
(51) Int. Cl.: A61B 17/29, A61B 90/00, A61B 17/00, A61B 17/30, A61B 17/12, A61M 1/00, A61B 17/42, A61N 1/05, A61N 1/36, A61B 17/34

(54) **BODY FLUID REGULATING APPARATUS**
VORRICHTUNG ZUR KÖRPERFLÜSSIGKEITSREGULIERUNG
APPAREIL DE RÉGULATION DE LIQUIDE CORPOREL

(30) Priority: 13.12.2022 US 202263387098 P
(43) Date of publication of application: 24.09.2025
(73) Proprietor: Resq Medical Ltd., 2069207 Yokneam (IL)
(72) Inventor: MAFRA, Ali, 1911500 Sulam (IL); STARK, Michael, 6997226 Tel Aviv (IL); DACH, Itamar, 3710302 Pardes Hanna (IL); WEICHSELBAUM, Amnon, 3220482 Haifa (IL)
(74) Representative: Jaeger, Michael David
(86) International application number: PCT/IB2023/062257
(87) International publication number: WO 2024/127158

(56) References cited:
- US-A1- 2004 215 215
- US-A1- 2005 055 043
- US-A1- 2019 069 929

## Description

### FIELD OF THE INVENTION

The present invention relates to a body fluid regulating apparatus, such as for treating postpartum hemorrhage.

### BACKGROUND OF THE INVENTION

Obstetric hemorrhage is the most common and dangerous complication of childbirth. Traditionally, postpartum hemorrhage (PPH) has been defined as greater than 500 mL estimated blood loss associated with vaginal delivery or greater than 1000 mL estimated blood loss associated with cesarean delivery. This was redefined by the American College of Obstetrics and Gynecology in 2017, and the current definition is cumulative blood loss greater than 1000 mL with signs and symptoms of hypovolemia within 24 hours of the birth process, regardless of the route of delivery. While this change was made with the knowledge that blood loss at the time of delivery is routinely underestimated, blood loss at the time of vaginal delivery greater than 500 mL should be considered abnormal with the potential need for intervention. Primary postpartum hemorrhage is bleeding that occurs in the first 24 hours after delivery, while secondary postpartum hemorrhage is characterized as bleeding that occurs 24 hours to 12 weeks postpartum.

There are several causes for PPH such as uterine atony, retained placenta, defects in coagulation and uterine inversion. The clinically most frequent cause is uterine atony, which results in inadequate contraction of the myometrial fibers and insufficient occlusion of the spiral arteries leading to uncontrolled hemorrhage.

US-A1-2019/069929 describes a postpartum balloon system including a flexible elongate catheter, an inflatable balloon, and a suction member attached to the distal end of the catheter. The system can be delivered into the uterus, where suction is applied to the suction member to anchor the balloon to the internal wall of the uterus. The catheter may include drainage ports both proximal and distal from the balloon. The system may include a stiffening stylet inserted into the drainage lumen of the catheter for aiding in delivery of the balloon. The stiffening stylet can be removed after placement of the balloon. The stiffening stylet can include side passageways that match the drainage ports and a lumen that allows for drainage through the stylet, as well as for administering drugs or other compounds through the drainage ports while the balloon is in place.

### SUMMARY

The present invention seeks to provide a body fluid regulating apparatus, such as for treating postpartum hemorrhage. Although the invention is described with reference to a uterine hemorrhage regulating apparatus, the invention can be used to regulate or stop other body fluids from other organs, such as the urinary bladder and other organs. The invention is defined by the appended claims. Any embodiments described herein that do not fall within the scope of the claims are provided for illustrative purposes only and do not form part of the claimed invention.

The apparatus includes means for seizing, grasping, and holding the uterine cavity tissue (endometrium and or myometrium) while applying negative pressure (vacuum) to the cavity, in order to evacuate blood clots and other debris from the uterus, and stop the bleeding by contracting the uterus and forcing contact between the walls of the uterus. Additional features include, without limitation, injection of various fluids or other substances into the uterus cavity or directly into the endometrium/ myometrium, such as substances that enhance uterine contractility.

The apparatus includes an anchoring inflatable plug or seal, such as a balloon. The inflatable plug, which may be fixed or slidable and adjustable, may be placed in the cervix or vagina. When expanded, the plug blocks the opening of the uterus, and thereby fixes the apparatus in place and allows a vacuum to be created in the uterine cavity.

The shaft of the apparatus includes an inner tube inside an outer tube with a gap between the two tubes. The inner tube has a smaller diameter than the outer tube and is formed with apertures that are larger in size than apertures formed on the outer tube. The apertures of the inner tube and of the outer tube may vary in size and shape.

The vacuum source (such as a vacuum pump) is coupled to the inner tube and creates a negative pressure in the inner tube which creates a pressure difference between the inner tube and the uterine walls, via the apertures in the inner tube, the gap between the tubes and the apertures in the outer tube. Thus, the negative pressure sucks the uterine walls towards the outer tube. The structure of tube in tube provides an optimal condition for maintaining the vacuum continuously. This is because the uterine walls press against and around the outer tube (portions of the uterine walls press against each other to help stop the bleeding), but do not press or abut against the inner tube, so that the vacuum in the inner tube is maintained.

The outer tube may have elongated ribs that divide the outer tube into different compartments. These ribs maintain a gap between the outer tube and the inner tube and prevent the outer tube from collapsing against the inner tube.

The structure of the inner tube and outer tube has another purpose in forming a blood clot filtering assembly. When the vacuum pump is activated, uterine debris, such as blood clots and tissue remnants, and blood are sucked through the apertures of the outer tube into the larger apertures of the inner tube. Debris which is larger than the apertures of the outer tube do not pass through the outer tube and remain on the outer tube or clog the apertures of the outer tube. As long as some apertures of the outer tube remain open, the vacuum originating in the inner tube continues to collect debris and to press the uterine walls against the outer tube and also against each other where there is no overlap with the outer tube. (If all apertures of the outer tube become clogged, the apparatus can be cleaned, such as with application of positive pressure to dislodge the clogs or removed and cleaned.) The debris and clots may be collected by the suction force into a collection vessel. This collected fluid may undergo filtration and treatment to be used for reinfusion.

The shape and size of the apertures may be selected for optimal suction of debris. For example, a cone shape (starting larger outwards to smaller inwards), or an irregular shape, may enable exerting negative pressure to suck in clotting debris. Apertures may be covered by or contain a mesh or mesh-like material which may cut or break up the clots, thereby minimizing the possibility for the clots to completely clog the apertures.

In a non-limiting embodiment of the apparatus, grasping members are provided which not only grasp the uterine tissue but also can be used to deliver one or more drugs or other substances into the endometrium, myometrium or uterine cavity. For example, an array of needles or rings containing curved and hidden hooks may be placed on the shaft of the device. Each ring may be able to deploy the hooks independently. After inserting the device into the uterus, inflating the anchoring balloon in the vagina or cervix, turning on the vacuum and collapsing the uterine tissue around the device, the grasping mechanism may be actuated, for example manually or by an actuation mechanism, allowing the hooks to be pulled out so that they become embedded in the adjacent uterine tissue (mechanical grasping). The graspers may fix the upper and lower walls in the correct position facing each other and prevent rotation and twisting of the tissue during vacuum activation. Drugs for enhancing uterine contractility can be delivered via the grasping members, in which the grasping members have a hollow lumen through which a drug can be injected. The grasping member may protrude in a manner which will optimize the relevant length for tissue penetration at this anatomical site.

Retractable grasping members may have a stopper to enable accurate depth penetration into the tissue.

In an embodiment of the apparatus, a conical structure may be added to the distal tip of the device, which may include fixation members that allow the conical structure to be affixed to the fundus tissue, such as by mechanical fixation which may be rotated to grasp the fundus and rotated in an opposite direction to release the fundus. The conical structure can also be attached to the fundus by a vacuum force, either the same vacuum source as the inner tube or a second vacuum source. These retractable fixation members can also serve for injection of drugs into the fundus tissue or other tissues for enhancing uterine contractility.

In an embodiment of the apparatus, an atraumatic tip may be provided at the end of the shaft (the shaft is the assembly of the inner and outer tubes) to minimize the possibility for tissue trauma. (The material from which the tubes are made may be flexible or semi-flexible, and may be inert, to further minimize the possibility for tissue trauma.) The atraumatic tip may also help in corrective repositioning of the uterine tissue, such as in the case of tissue inversion. The tip may include one or more apertures to enable distal suction of fluid and debris.

In a non-claimed example, a device comprising forceps arms may be provided, which is inserted into the uterine cavity. Inside the uterus, the forceps arms are opened until making physical contact with the anterior and posterior uterine walls. The arms may be attached to the uterine walls by using both vacuum and grasping. Closing of the forceps causes the uterine anterior and posterior walls to fold and/or move towards each other, thereby leading to a forced contraction of the uterus and cessation of the bleeding. Afterwards the device may be removed.

### BRIEF DESCRIPTION OF DRAWINGS

The present invention will be understood and appreciated more fully from the following detailed description, taken in conjunction with the drawings in which:
Fig. 1 is a simplified illustration of a body fluid regulating apparatus, in accordance with a non-limiting embodiment of the present invention.
Fig. 2 is a more detailed illustration of the proximal portion of the body fluid regulating apparatus.
Fig. 3 is a more detailed illustration of the inflatable plug of the body fluid regulating apparatus.
Fig. 4 is a more detailed illustration of the distal portion of the body fluid regulating apparatus.
Fig. 5 is a more detailed illustration of one type of grasping member of the body fluid regulating apparatus, which is a needle through which a drug can be delivered.
Fig. 5A is a simplified illustration of an outer tube which has a longitudinal gap (that is, the outer tube does not have a full 360° perimeter), in accordance with a non-limiting embodiment of the invention.
Figs. 6A and 6B are side-view and end-view illustrations, respectively, of another type of grasping member of the body fluid regulating apparatus, which includes a ring around the outer tube which has needles through which a drug can be delivered.
Fig. 7 is a simplified illustration of a conical structure coupled to the distal tip of the body fluid regulating apparatus, which includes fixation members that allow the conical structure to be affixed to the fundus tissue.
Figs. 8A-8E are simplified illustrations of a body fluid regulating apparatus, in accordance with another non-limiting embodiment of the apparatus.
Fig. 9 is a detailed illustration of the distal portion of the body fluid regulating apparatus, showing stimulating electrodes and radiopaque markers.
Figs. 10A and 10B are simplified illustrations of internal ribs in the outer tube.

### DETAILED DESCRIPTION

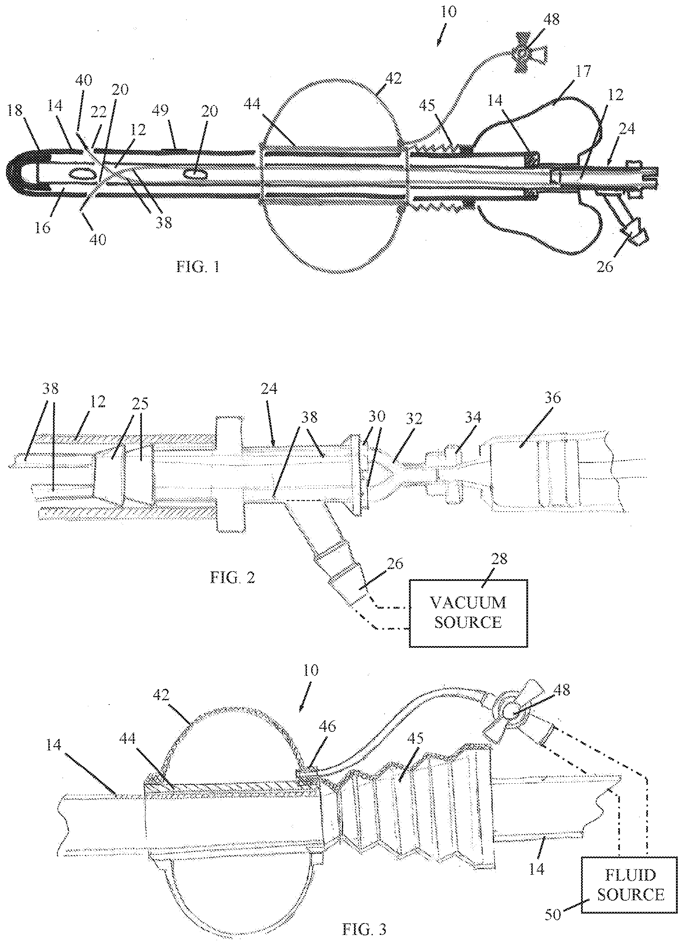

Reference is now made to Fig. 1, which illustrates a body fluid regulating apparatus 10, in accordance with a non-limiting embodiment of the present invention. In the following description, embodiments that do not include all the features of the apparatus as defined in the claims are presented for illustrative purposes only and do not form part of the presently claimed invention.

The apparatus 10 may include an inner tube 12 inside an outer tube 14 (the two tubes together are also called the intrauterine shaft or simply shaft) with a gap 16 between the two tubes. A distal inner cap 18 may be mounted at the distal ends of inner tube 12 and outer tube 14, wherein cap 18 is located in gap 16 and rests over inner tube 12 and outer tube 14 (such as, over the outer surface of inner tube 12 and the inner or outer surface of outer tube 14). Cap 18 thus helps maintain patency of gap 16 and serves as a protective frontal dome. The apparatus 10 may include a proximal handle 17.

Gap 16 and the lumen of the internal tube 12 may be used as a working channel for tools, such as but not limited to, fiber optic tools, hysteroscopy tools, graspers, biopsy tools and more.

The inner tube 12 has a smaller diameter than the outer tube 14 and is formed with apertures 20 that are larger in size than apertures 22 formed in the outer tube 14. The apertures 20 and 22 may be of any size and shape, such as but not limited to, circular, elliptical, polygonal, regular, irregular, etc. For example, without limitation, the larger apertures 20 may be oval in the range of 2-6 mm in width and 5-10 mm in length; the smaller apertures 22 may be oval in the range of 1-5 mm in width and 3-7 mm in length The apertures 20 and 22 may be formed in any pattern around the circumference, and along the length, of inner tube 12 and outer tube 14, respectively.

Optionally, outer tube 14 may be a mesh or porous covering or other kinds of covering with one or more apertures and the term "outer tube" encompasses all of these structures.

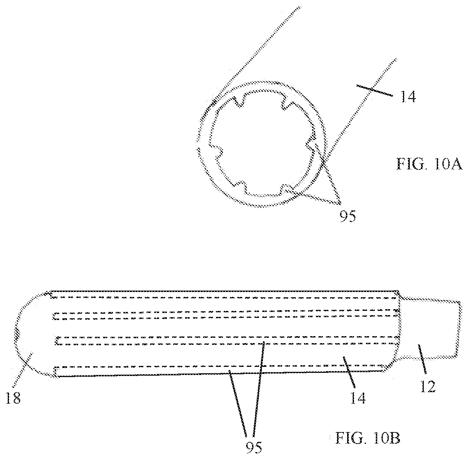

Referring to Figs. 10A and 10B, it is seen that outer tube 14 may have elongated ribs 95 that divide the outer tube into different compartments. Ribs 95 are positioned on an inner surface of outer tube 14 between the apertures so they do not interfere with the apertures. Ribs 95 maintain a gap between the outer tube and the inner tube and prevent the outer tube from collapsing against the inner tube.

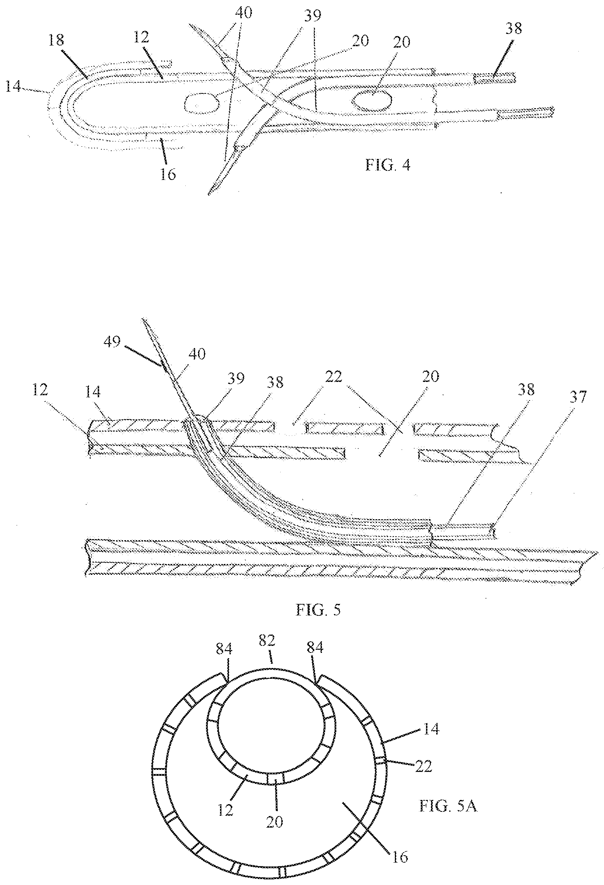

One of such exemplary structures is now described with reference to Fig. 5A. In this embodiment, the outer tube 14 has a longitudinal gap 82 (that is, the outer tube does not have a full 360° perimeter). The edges of outer tube 14 along the longitudinal gap 82 may be provided with seals 84 that sealingly abut against the outer contour of inner tube 12 to maintain the vacuum in inner tube 12.

Reference is now made additionally to Fig. 2, which illustrates the proximal portion of the body fluid regulating apparatus 10. For simplicity, outer tube 14 and handle 17 are not shown in Fig. 2. A fluid adaptor 24 is sealingly coupled to the proximal portion of inner tube 12, such as with one or more seals 25. It is noted that the term "fluid" encompasses liquid and/or gas. Fluid adaptor 24 may include a vacuum port 26 which can introduce negative pressure into the inner lumen of inner tube 12 from a vacuum source 28.

Fluid adaptor 24 (any kind of fluid inlet or connector) may include one or more catheter ports 30. In the non-limiting illustrated embodiment, there are two catheter ports 30 proximally coupled to a bifurcated manifold 32, a proximal portion of which is coupled to a fluid connector 34, such as a Luer connector. A syringe 36 may be coupled to fluid connector 34. The catheter ports 30 are coupled to one or more catheters 38 (in the non-limiting illustrated embodiment, there are two catheters 38. It is noted that in Fig. 2 catheter 38 is not connected to vacuum port 26 or vacuum source 28). Referring again to Fig. 1, the apparatus 10 may include one or more grasping members 40, which in this embodiment, are needles coupled to catheters 38. As will be described more in detail below, syringe 36 (Fig. 2) can introduce drugs via catheters 38 to the hollow lumens of grasping members 40 for introducing drugs into the uterine tissue. The term "needle" as used throughout the description and claims encompasses a needle, hook, lance, barb and any other sharp-tipped element.

Reference is now made to Fig. 3 (for simplicity inner tube 12 is not shown in Fig. 3). Apparatus 10 may include an anchoring inflatable plug 42, such as a balloon. The inflatable plug 42 may be coupled to a collar 44, which can adjustably slide along outer tube 14. A flexible member 45, such as a bellows, may be coupled to the proximal portion of inflatable plug 42 and to outer tube 14 to allow for flexibility in the position of inflatable plug 42. Alternatively, inflatable plug 42 may be fixedly coupled to outer tube 14. When expanded, inflatable plug 42 blocks the opening of the uterus, and thereby fixes apparatus 10 in place and allows a vacuum to be created in the uterine cavity.

The inflatable plug 42 may include a balloon fluid port 46 which is coupled via a valve or stopcock 48 to a fluid source 50. In this manner, inflatable plug 42 can be inflated by introducing therein fluid and can be deflated by removing therefrom fluid. Valve 48 may be embedded inside the device.

Figs. 4 and 5 are more detailed illustrations of the distal portion of the body fluid regulating apparatus that shows the grasping members 40 comprising needles coupled to catheters 38. The catheters 38 may have covered by sheaths 39. The inner lumen 37 (Fig. 5) of the catheter 38 is the passageway for introducing, for example, a drug to the inner lumen of the needle for drug delivery to the uterine tissue. Without limitation, the drug may be a uterotonic agent, oxytocin, or drugs used to enhance uterine contractility, such as but not limited to, ergot alkaloids and prostaglandins, such as metheargine, carboprost and dinoprostone and misoprostol.

As seen in Fig. 5, a fiducial mark 49 may be disposed on grasping member 40 to help the surgeon visualize the spatial and rotation orientation of grasping member 40. Alternatively, fiducial mark 49 may be disposed on other portions of apparatus 10, such as but not limited to, a portion of outer tube 14 (as seen in Fig. 1). The fiducial marks may assist the user to control the orientation of the needles or other portions of the device, and to direct the needles to the upper and lower walls of the uterus.

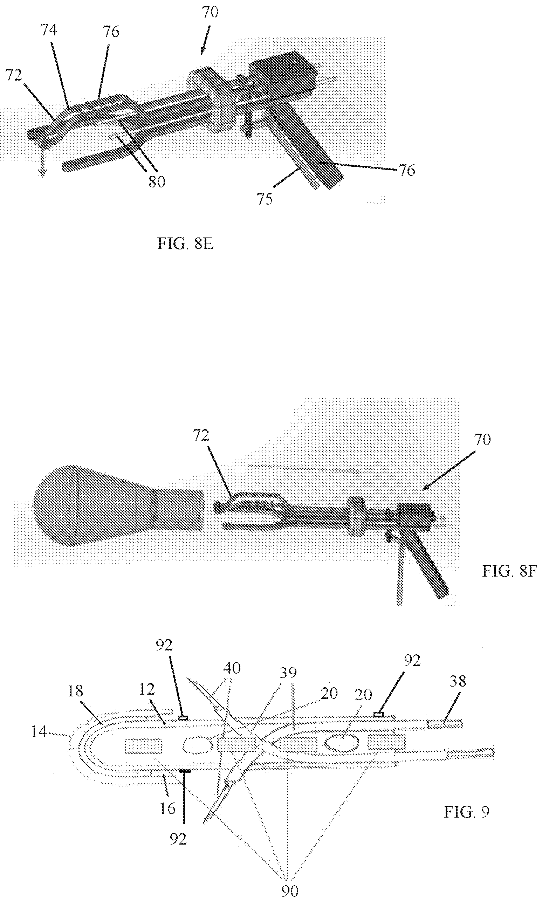

Another version of the body fluid regulating apparatus of Fig. 4 is shown in Fig. 9. In order to stimulate muscle contraction, one or more stimulating electrodes 90 may be positioned along the outer tube 14 (shaft) of the device (or any other suitable portion of the device). Electrodes 90 are positioned to come into contact with the organ tissue after the negative pressure (vacuum) is operated. Electrodes 90 stimulate muscle contraction by means of electrical impulses (electrical muscle stimulation or electromyostimulation). Electrodes 90 may be powered by a battery integrated in the device and activated when the procedure begins.

Fig. 9 also shows another optional feature of the invention, namely, one or more radiopaque markers 92 (for example, to help visualize the device using ultrasound, X-ray etc.), such as but not limited to, stripes, rings, bands and the like, which may be positioned on the outer tube 14 (shaft) of the device (or any other suitable portion of the device).

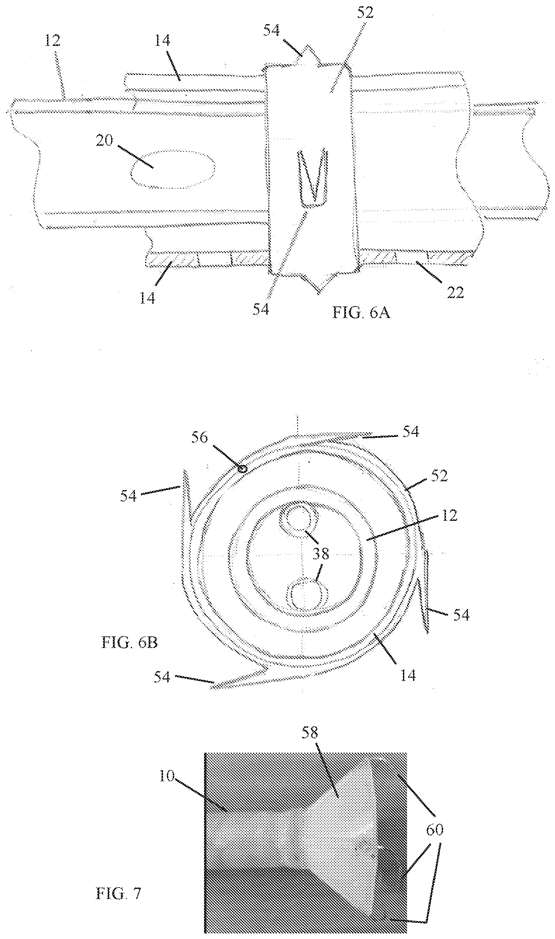

Reference is now made to Figs. 6A and 6B, which illustrate another type of grasping member of the body fluid regulating apparatus. In this embodiment, the grasping member includes a ring 52 around the outer tube 14 which has hooks, needles or lancets 54 protruding outwards from ring 52. Needles 54 may be optionally coupled to catheters 38 so that drug can be delivered therethrough. One or more sensors 56 may be coupled to needles 54 to provide an indication of whether the needle is engaged in tissue or not. The sensor 56 may be, without limitation, a contact sensor (e.g., capacitance which changes if contacting the tissue or not), pH sensor (pH changes if contacting the tissue or not), vacuum sensor, or other sensors.

In use of the apparatus, the vacuum source (such as a vacuum pump) is coupled to the inner tube and creates a negative pressure in the inner tube which creates a pressure difference between the inner tube and the uterine walls, via the apertures in the inner tube, the gap between the tubes and the apertures in the outer tube. Thus, the negative pressure sucks the uterine walls towards the outer tube. The structure of tube in tube provides an optimal condition for maintaining the vacuum continuously. This is because the uterine walls press against and around the outer tube (portions of the uterine walls press against each other to help stop the bleeding), but do not press or abut against the inner tube, with or without the support of the elongated ribs that keep the gap between the outer and inner tubes so that the vacuum in the inner tube is maintained. After inserting the device into the uterus, inflating the anchoring balloon in the vagina, turning on the vacuum and collapsing the uterine tissue around the device, the grasping members may be manually or automatically activated, allowing the hooks or needles to be pulled out so that they become embedded in the adjacent uterine tissue (mechanical grasping). Drugs for enhancing uterine contractility can be delivered via the grasping members, in which the grasping members have a hollow lumen through which a drug can be injected.

The structure of the inner tube and outer tube has another purpose in forming a blood clot filtering assembly. When the vacuum pump is activated, uterine debris, such as blood clots and tissue remnants, and blood are sucked through the apertures of the outer tube into the larger apertures of the inner tube. Debris which is larger than the apertures of the outer tube do not pass through the outer tube and remain on the outer tube or clog the apertures of the outer tube. As long as some apertures of the outer tube remain open, the vacuum originating in the inner tube continues to collect debris and to press the uterine walls against the outer tube. (If all apertures of the outer tube become clogged, the apparatus can be cleaned (either while in place or after removal), such as with application of positive pressure to dislodge the clogs.) The debris may be collected by the suction force into a collection vessel. This collected blood may undergo filtration and treatment to be used for reinfusion.

Reference is now made to Fig. 7. Optionally, a conical structure 58 may be coupled to the distal tip of apparatus 10, which may include fixation members 60 (e.g., hooks or needles and such) that allow the conical structure 58 to be affixed to the fundus tissue, such as by mechanical fixation in which the fixation members 60 may be retractable or rotated to grasp the fundus and rotated in an opposite direction to release the fundus. The conical structure 58 can also be attached to the fundus by a vacuum force, either the same vacuum source as the inner tube or a second vacuum source.

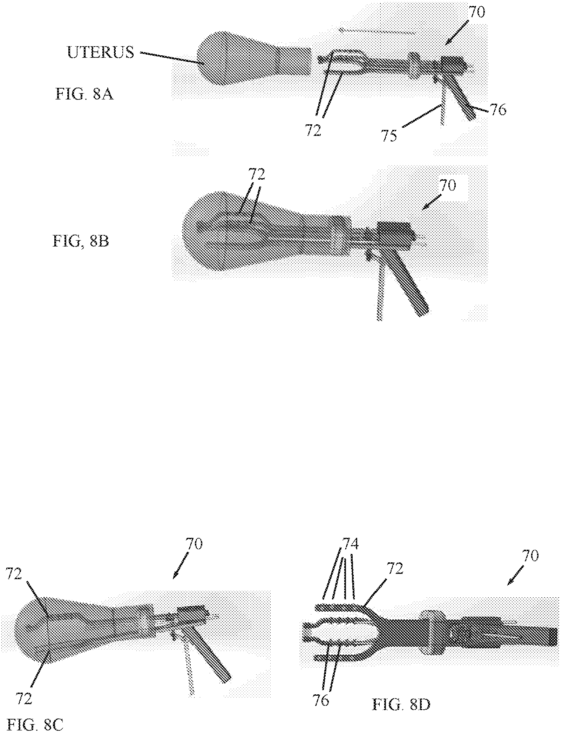

Reference is now made to Figs. 8A-8E, which illustrate a body fluid regulating apparatus 70, in accordance with another non-limiting embodiment of the present invention. In a non-claimed example, a body fluid regulating apparatus 70 may include two or more forceps arms. 72. Each forceps arm 72 includes vacuum ports 74 (Fig. 8D) which are coupled to a vacuum source as in the embodiment of Fig. 1. The forceps arms 72 may be coupled to a trigger mechanism 75 provided on a handle 76. The forceps arms 72 may be moved radially inwards and outwards by the trigger mechanism 75. Each forceps arm 72 may also include grasping members 78 (Fig. 8D), such as needles or hooks.

Figs. 8A and 8B show the apparatus 70 before and after insertion into the uterus, respectively.

Inside the uterus, the forceps arms are opened until making physical contact with the anterior and posterior uterine walls. As seen in Fig. 8C, the lower forceps arm 72 may be attached by vacuum to the lower uterine wall (posterior wall) and the upper forceps arm 72 may be attached by vacuum to the upper uterine wall (anterior wall). The arms may be attached to the uterine walls by using both vacuum and grasping.

As seen in Fig. 8E, closing of the forceps causes the uterine anterior and posterior walls to move towards each other, thereby leading to a forced contraction of the uterus and cessation of the bleeding. Afterwards the device may be removed (grasper released and device removed), as seen in Fig. 8F.

As seen in Fig. 8E, the apparatus may include one or more flow sensor probes 80 for measuring the level of bleeding in real time.

## Claims

1. A body fluid regulating apparatus (10) comprising:
an inner tube (12) disposed inside an outer tube (14) with a gap (16) between said inner and outer tubes (12, 14), said inner tube (12) being formed with apertures (20) that are larger in size than apertures (22) formed in said outer tube (14);
a fluid adaptor (24) sealingly coupled to a proximal portion of said inner tube (12), said fluid adaptor (24) comprising a vacuum port (26) adapted to be coupled to a vacuum source (28) for creating a negative pressure in said inner tube (12), wherein said outer tube (14) is adapted for uterine tissue to press thereagainst; and
an inflatable plug (42) disposed on said outer tube (14).

2. The body fluid regulating apparatus (10) according to claim 1, further comprising grasping members (40) arranged to protrude outwards of said outer tube (14).

3. The body fluid regulating apparatus (10) according to claim 1 or claim 2, wherein said fluid adaptor (24) comprises one or more catheter ports (30) coupled to one or more catheters (38) disposed in said inner tube (12), and grasping members (40) are coupled to distal portions of said one or more catheters (38).

4. The body fluid regulating apparatus (10) according to claim 3, wherein said grasping members (40) are arranged to protrude outwards of said outer tube (14).

5. The body fluid regulating apparatus (10) according to claim 2, wherein said grasping members (40) comprise needles.

6. The body fluid regulating apparatus (10) according to claim 3, wherein said grasping members (40) comprise hollow needles and a drug and other materials is flowable through said one or more catheters (38) and said hollow needles.

7. The body fluid regulating apparatus (10) according to any one of claims 1-6, wherein said inflatable plug (42) is slidable along said outer tube (14).

8. The body fluid regulating apparatus (10) according to any one of claims 2-6, further comprising a fiducial mark (49) for indicating spatial and rotation orientation of said grasping members (40).

9. The body fluid regulating apparatus (10) according to any one of claims 2-6 or claim 8, wherein said grasping members (40) are disposed around said outer tube (14).

10. The body fluid regulating apparatus (10) according to any one of claims 1-9, wherein a cap (18) is mounted at distal ends of said inner tube (12) and said outer tube (14), wherein said cap (18) is located in said gap (16) and rests over said inner tube (12) and said outer tube (14).

11. The body fluid regulating apparatus (10) according to any one of claims 2-10, further comprising one or more sensors (56) adapted to provide an indication of whether said grasping members (40) are engaged in tissue or not.

12. The body fluid regulating apparatus (10) according to any one of claims 2-11, further comprising one or more stimulating electrodes (90) operative to stimulate muscle contraction by means of electrical impulses.

13. The body fluid regulating apparatus (10) according to any one of claims 2-12, further comprising one or more radiopaque markers (92).

14. The body fluid regulating apparatus (10) according to any one of claims 2-13, further comprising one or more elongated ribs (95) formed on an inner surface of said outer tube (14).

## Patentansprüche

1. Vorrichtung (10) zum Regulieren von Körperflüssigkeiten, die Folgendes umfasst:
einen Innenschlauch (12), der mit einem Spalt (16) zwischen Innen- und Außenschlauch (12, 14) in einem Außenschlauch (14) angeordnet ist, wobei der Innenschlauch (12) mit Öffnungen (20) ausgebildet ist, die größer sind als in dem Außenschlauch (14) ausgebildete Öffnungen (22);
einen Flüssigkeitsadapter (24), der dicht mit einem proximalen Abschnitt des Innenschlauchs (12) gekoppelt ist, wobei der Flüssigkeitsadapter (24) einen Vakuumanschluss (26) umfasst, der so ausgelegt ist, dass er zum Erzeugen eines Unterdrucks in dem Innenschlauch (12) mit einer Vakuumquelle (28) gekoppelt werden kann, wobei der Außenschlauch (14) so ausgelegt ist, dass Gebärmuttergewebe dagegendrücken kann; und
einen aufblasbaren Tampon (42), der an dem Außenschlauch (14) angeordnet ist.

2. Vorrichtung (10) zum Regulieren von Körperflüssigkeiten nach Anspruch 1, die ferner Greifelemente (40) umfasst, welche so angeordnet sind, dass sie aus dem Außenschlauch (14) herausragen.

3. Vorrichtung (10) zum Regulieren von Körperflüssigkeiten nach Anspruch 1 oder 2, wobei der Flüssigkeitsadapter (24) einen oder mehrere Katheteranschlüsse (30) umfasst, die mit einem oder mehreren in dem Innenschlauch (12) angeordneten Kathetern (38) gekoppelt sind, und Greifelemente (40) mit distalen Abschnitten des einen oder der mehreren Katheter (38) gekoppelt sind.

4. Vorrichtung (10) zum Regulieren von Körperflüssigkeiten nach Anspruch 3, wobei die Greifelemente (40) so angeordnet sind, dass sie aus dem Außenschlauch (14) herausragen.

5. Vorrichtung (10) zum Regulieren von Körperflüssigkeiten nach Anspruch 2, wobei die Greifelemente (40) Nadeln umfassen.

6. Vorrichtung (10) zum Regulieren von Körperflüssigkeiten nach Anspruch 3, wobei die Greifelemente (40) Kanülen umfassen und ein Medikament und anderes Material durch den einen oder die mehreren Katheter (38) und die Kanülen geleitet werden kann.

7. Vorrichtung (10) zum Regulieren von Körperflüssigkeiten nach einem der Ansprüche 1-6, wobei der aufblasbare Tampon (42) an dem Außenschlauch (14) entlang verschiebbar ist.

8. Vorrichtung (10) zum Regulieren von Körperflüssigkeiten nach einem der Ansprüche 2-6, die ferner eine Referenzmarkierung (49) zum Anzeigen einer räumlichen und rotationsbezogenen Ausrichtung der Greifelemente (40) umfasst.

9. Vorrichtung (10) zum Regulieren von Körperflüssigkeiten nach einem der Ansprüche 2-6 oder Anspruch 8, wobei die Greifelemente (40) um den Außenschlauch (14) herum angeordnet sind.

10. Vorrichtung (10) zum Regulieren von Körperflüssigkeiten nach einem der Ansprüche 1-9, wobei an distalen Enden des Innenschlauchs (12) und des Außenschlauchs (14) eine Kappe (18) angebracht ist, wobei sich die Kappe (18) in dem Spalt (16) befindet und auf dem Innenschlauch (12) und dem Außenschlauch (14) aufliegt.

11. Vorrichtung (10) zum Regulieren von Körperflüssigkeiten nach einem der Ansprüche 2-10, die ferner einen oder mehrere Sensoren (56) umfasst, welche so ausgelegt sind, dass sie eine Angabe dazu liefern, ob die Greifelemente (40) am Gewebe anliegen.

12. Vorrichtung (10) zum Regulieren von Körperflüssigkeiten nach einem der Ansprüche 2-11, die ferner eine oder mehrere Stimulationselektroden (90) umfasst, welche dazu dienen, mithilfe von elektrischen Impulsen eine Muskelkontraktion anzuregen.

13. Vorrichtung (10) zum Regulieren von Körperflüssigkeiten nach einem der Ansprüche 2-12, die ferner einen oder mehrere strahlungsundurchlässige Marker (92) umfasst.

14. Vorrichtung (10) zum Regulieren von Körperflüssigkeiten nach einem der Ansprüche 2-13, die ferner eine oder mehrere längliche Rippen (95) umfasst, welche an einer Innenfläche des Außenschlauchs (14) ausgebildet sind.

## Revendications

1. Appareil de régulation de fluide corporel (10) comprenant :
un tube interne (12) disposé à l'intérieur d'un tube externe (14) avec un écartement (16) entre lesdits tubes interne et externe (12, 14), ledit tube interne (12) étant formé avec des ouvertures (20) qui sont plus grandes en taille que des ouvertures (22) formées dans ledit tube externe (14) ;
un adaptateur de fluide (24) couplé de manière étanche à une partie proximale dudit tube interne (12), ledit adaptateur de fluide (24) comprenant un orifice de vide (26) conçu pour être couplé à une source de vide (28) pour créer une pression négative dans ledit tube interne (12), dans lequel ledit tube externe (14) est conçu pour que le tissu utérin exerce une pression contre lui ; et
un bouchon gonflable (42) disposé sur ledit tube externe (14).

2. Appareil de régulation de fluide corporel (10) selon la revendication 1, comprenant en outre des éléments de préhension (40) agencés pour faire saillie vers l'extérieur dudit tube externe (14).

3. Appareil de régulation de fluide corporel (10) selon la revendication 1 ou la revendication 2, dans lequel ledit adaptateur de fluide (24) comprend un ou plusieurs orifices de cathéter (30) couplés à un ou plusieurs cathéters (38) disposés dans ledit tube interne (12), et des éléments de préhension (40) sont couplés aux parties distales desdits un ou plusieurs cathéters (38).

4. Appareil de régulation de fluide corporel (10) selon la revendication 3, dans lequel lesdits éléments de préhension (40) sont agencés pour faire saillie vers l'extérieur dudit tube externe (14).

5. Appareil de régulation de fluide corporel (10) selon la revendication 2, dans lequel lesdits éléments de préhension (40) comprennent des aiguilles.

6. Appareil de régulation de fluide corporel (10) selon la revendication 3, dans lequel lesdits éléments de préhension (40) comprennent des aiguilles creuses et un médicament et d'autres matériaux peuvent s'écouler à travers lesdits un ou plusieurs cathéters (38) et lesdites aiguilles creuses.

7. Appareil de régulation de fluide corporel (10) selon l'une quelconque des revendications 1 à 6, dans lequel ledit bouchon gonflable (42) est capable de coulisser le long dudit tube externe (14).

8. Appareil de régulation de fluide corporel (10) selon l'une quelconque des revendications 2 à 6, comprenant en outre une marque de repère (49) destinée à indiquer une orientation spatiale et de rotation desdits éléments de préhension (40).

9. Appareil de régulation de fluide corporel (10) selon l'une quelconque des revendications 2 à 6 ou 8, dans lequel lesdits éléments de préhension (40) sont disposés autour dudit tube externe (14).

10. Appareil de régulation de fluide corporel (10) selon l'une quelconque des revendications 1 à 9, dans lequel un capuchon (18) est monté au niveau des extrémités distales dudit tube interne (12) et dudit tube externe (14), dans lequel ledit capuchon (18) est situé dans ledit écartement (16) et repose sur ledit tube interne (12) et ledit tube externe (14).

11. Appareil de régulation de fluide corporel (10) selon l'une quelconque des revendications 2 à 10, comprenant en outre un ou plusieurs capteurs (56) conçus pour fournir une indication du fait que lesdits éléments de préhension (40) sont entrés en prise dans le tissu ou non.

12. Appareil de régulation de fluide corporel (10) selon l'une quelconque des revendications 2 à 11, comprenant en outre une ou plusieurs électrodes de stimulation (90) fonctionnelle pour stimuler la contraction musculaire au moyen d'impulsions électriques.

13. Appareil de régulation de fluide corporel (10) selon l'une quelconque des revendications 2 à 12, comprenant en outre un ou plusieurs marqueurs radio-opaques (92).

14. Appareil de régulation de fluide corporel (10) selon l'une quelconque des revendications 2 à 13, comprenant en outre une ou plusieurs nervures allongées (95) formées sur une surface interne dudit tube externe (14).
